# EUROPEAN PATENT APPLICATION

(11) **EP 4 421 085 A1**
(43) Date of publication of application: **28.08.2024**
(21) Application number: 23158879.9
(22) Date of filing: 27.02.2023
(51) Int. Cl.: C07K 14/435, A61K 38/17, A61P 25/28

(54) **ALPHA SYNUCLEIN-BINDING POLYPEPTIDE**

(71) Applicant: Amylonix AB, 121 36 Johanneshov (SE)
(72) Inventor: LÖFBLOM, John, 146 54 Tullinge (SE); STÅHL, Stefan, 111 40 Stockholm (SE); LINDBERG, Hanna, 116 22 Stockholm (SE); HJELM, Linnea, 170 70 Solna (SE)
(74) Representative: Kransell & Wennborg KB

(57) **Abstract**

There is provided an alpha synuclein-binding polypeptide comprising a first and a second subunit and a linker linking the C-terminus of the first subunit to the N-terminus of the second subunit.

## Description

### TECHNICAL FIELD

The present disclosure relates to the field of treating or preventing Parkinson's disease and Lewy body dementia.

### BACKGROUND

Neurodegenerative disorders (NDs) are a family of diseases where the degradation of neurons impacts the cognitive and motor function, and examples include Alzheimer's disease (AD), Frontotemporal Dementia (FTD), Amyotrophic Lateral Sclerosis (ALS), and Parkinson's Disease (PD). The second most common ND is Parkinson's disease (PD), associated with several motor- and non-motor symptoms. Alpha synuclein is a neuronal protein and the main constituent of Lewy bodies which is a neuropathological hallmark of several dementias such as Parkinson's disease and Lewy body dementia.

During the progression of PD, neurons in the substantia nigra become damaged and eventually dies, causing a decrease in the production of dopamine. Lower dopamine level leads to difficulties in controlling movements and the typical PD symptoms (Maiti et al. 2017). Toxicity caused by overexpression, or mutations in the alpha synuclein (aSN) gene yielding a higher aggregation propensity or secretion propensity correlates to the neuronal degeneration (Breydo, Wu, and Uversky 2012; Fornai et al. 2004; Guan et al. 2020). Today, drugs for PD only slow the progression and no curative treatments for PD are available (McFarthing et al. 2022). However, there are several disease-modifying drugs emerging. Two prominent examples are the monoclonal antibody Prasinezumab that targets soluble and aggregated aSN (Pagano et al. 2022; Schenk et al. 2017), and the neuroprotective peptide Liraglutide (Victoza, repurposed from diabetes treatment) (Liu et al. 2015). Several other antibodies are in development for therapeutic and diagnostic purposes for PD (McFarthing et al. 2022). However, to obtain a smaller molecular size and easily introduce multi specificity, antibody derivatives and engineerable small protein scaffolds have been suggested as interesting alternatives (Löfblom, Frejd, and Stahl 2011; Stahl et al. 2017; Zhong and D'Antona 2021).

A new scaffold protein has been designed based on a dimeric type of affibody molecules, which was first reported for the amyloid-beta binder Z_{Aβ3} (Grönwall et al. 2007). The scaffold was engineered into a head-to-tail genetic dimer, truncated in the N-terminal and a new flexible serine and glycine linker was introduced between the two subunits. Based on the sequences and structures of previous dimeric affibody molecules in complex with aggregation prone peptides, eleven positions in each subunit were selected for randomization to construct a new combinatorial library. The new dimeric scaffold was denoted *Sequestrin* and in a proof-of-concept study intended to assess the quality of the scaffold and library design, new high affinity sequestrins against amyloid beta peptide were selected using phage display technology (Hjelm et al. 2023).

### SUMMARY

The present disclosure describes use of the new sequestrin library in phage display selections against aSN with the aim to generate high-affinity aggregation inhibitors. Sequestrins are a new type of small affinity proteins with a mode-ofbinding similar to protein chaperones, shielding the hydrophobic parts of neurotoxic proteins and peptides and thereby acting as anti-amyloid agents. Upon binding, sequestrins co-fold with the neurotoxic peptide, forming a four-stranded beta-sheet with a secondary structure that have similarities to the beta-sheet rich structures seen in amyloid fibrils.

Characterization of the clones from the selection has revealed several binders with affinities in the low nanomolar range. Spectroscopy studies on secondary structure content has demonstrated structural rearrangements in the sequestrins as well as in aSN. Aggregation of wildtype aSN and three familial PD variants (A30P, E46K and A53T) (Maiti et al. 2017) was completely inhibited by several of the new sequestrins when mixed in equimolar concentrations.

The best binders demonstrated affinities around 10 nM and structural rearrangements in both sequestrin and alpha synuclein upon binding. Studying the effects of sequestrins on alpha synuclein *in vitro* demonstrated that the tested variants completely inhibited alpha synuclein aggregation at equimolar concentrations, including the A30P, E46K and A53T mutants that are linked with familial forms of Parkinson's disease and Lewy body dementia.

Based on the sequences of the identified high affinity binders (and the inventors' understanding of the general structure of the sequesterins), there is provided an alpha synuclein-binding polypeptide comprising a first and a second subunit and a linker linking the C-terminus of the first subunit to the N-terminus of the second subunit, wherein:
the first subunit comprises the amino acid sequence 
GX3X4X5X6X7X8X9X10X11X12X13X14X15X16CX18FIX21X22X23X24X25X26X27X28X29X30AX32
X33X34X35X36X37X38X39X40X41X42X43X44 (SEQ ID NO:45);
 and
the second subunit comprises the amino acid sequence 
X₆₂GX₆₄X₆₅VX₆₇X₆₈X₆₉X₇₀X₇₁X₇₂X₇₃X₇₄X₇₅X₇₆CX₇₈FX₈₀X₈₁X₈₂X₈₃X₈₄X₈₅X₈₆X₈₇X₈₈X₈₉
X₉₀AX₉₂X₉₃LX₉₅X₉₆X₉₇X₉₈X₉₉X₁₀₀X₁₀₁X₁₀₂X₁₀₃X₁₀₄ (SEQ ID NO:46),
and wherein
X₃ is G, R, P or K, preferably G or R, more preferably G,
X₄ is E, D, N or Q, preferably E,
X₅ is any amino acid except C, preferably R, P, L, T or E,
X₆ is any amino acid except C, preferably I, V or F,
X₇ is any amino acid except C, preferably Y, V, S or T,
X₈ is L or M, preferably L,
X₉ is P, T, R, L, Y, G or H, preferably P,
X₁₀ is N, E, D or Q, preferably N,
X₁₁ is L, A, I, V or G, preferably L,
X₁₂ is N or K, preferably N,
X₁₃ is A or P, preferably A,
X₁₄ is D, H, S, A or E, preferably D,
X₁₅ is Q, D, N or E, preferably Q,
X₁₆ is L or I, preferably L,
X₁₈ is A, I, V, G or L, preferably A or L, more preferably A,
X₂₁ is R, Q, N, H or K, preferably R,
X₂₂ is S, T, I, L or V, preferably S,
X₂₃ is L, A or V, preferably A,
X₂₄ is E, Q, D or N, preferably E or Q, more preferably E,
X₂₅ is D or E, preferably D,
X₂₆ is D or E, preferably D,
X₂₇ is P, R, Q, E, S or G, preferably P,
X₂₈ is S or T, preferably S,
X₂₉ is Q or N, preferably Q,
X₃₀ is S, Q, E, N or P, preferably S,
X₃₂ is N or Q, preferably N,
X₃₃ is L or I, preferably L,
X₃₄ is L or V, preferably L,
X₃₅ is A, I or L, preferably A,
X₃₆ is E or D, preferably E,
X₃₇ is A, I or L, preferably A,
X₃₈ is K or R, preferably K,
X₃₉ is K or R, preferably K,
X₄₀ is L, A, I or V, preferably L,
X₄₁ is N or Q, preferably N,
X₄₂ is D or E, preferably D,
X₄₃ is A, I or L, preferably A,
X₄₄ is Q or N, preferably Q,
X₆₂ is G or P, preferably G,
X₆₄ is E or D, preferably E,
X₆₅ is any amino acid except C, preferably P, R, T, I, V or A, more preferably P, R or T,
X₆₇ is any amino acid except C, preferably Y, L, W or M, more preferably Y or L,
X₆₈ is L or M, preferably L,
X₆₉ is P, L, T, R, L, Y or H, preferably P,
X₇₀ is N, E, D or Q, preferably N,
X₇₁ is L, A, I, V or G, preferably L,
X₇₂ is N or K, preferably N,
X₇₃ is A or P, preferably P,
X₇₄ is D, H, S or A, preferably D,
X₇₅ is Q or N, preferably Q,
X₇₆ is W or L, preferably W,
X₇₈ is A, I, V, G or L, preferably A,
X₈₀ is L, W, F, I or M, preferably L, W, For M, more preferably W or F, most preferably W,
X₈₁ is R, Q, N, H or K, preferably R,
X₈₂ is S, T, I, L or V, preferably S,
X₈₃ is L or A, preferably L,
X₈₄ is E, Q, D or N, preferably E,
X₈₅ is D or E, preferably D,
X₈₆ is D or E, preferably D,
X₈₇ is P, R, Q, E, S or G, preferably P,
X₈₈ is S or T, preferably S,
X₈₉ is Q or N, preferably Q,
X₉₀ is S, Q, E, N or P, preferably S,
X₉₂ is N or Q, preferably N,
X₉₃ is L or I, preferably L,
X₉₅ is A, I or L, preferably A,
X₉₆ is E or D, preferably E,
X₉₇ is A, I or L, preferably A,
X₉₈ is K or R, preferably K,
X₉₉ is K or R, preferably K,
X₁₀₀ is L, A, I or V, preferably L,
X₁₀₁ is N or Q, preferably N,
X₁₀₂ is D or E, preferably D,
X₁₀₃ is A, I or L, preferably A, and
X₁₀₄ is Q or N, preferably Q.

### BRIEF DESCRIPTION OF THE FIGURES

Figure 1a: Illustration of the randomization behind the library from which the phage display selection of the Examples section below was made. Group I: all amino acids (aa) except C. Group II: K or N, and P or A. Group III: hydrophobic aa. Group IV: all aa except P, G and C.
Figure 1b: Sequence of the sequestrin library with randomised positions marked with X. Mutations in randomized positions for five selected sequestrins.
Figure 1c: Alignment of sequestrins identified in the phage display experiment described in the Examples section below and the prior art sequence AS69 (also an aSN binder, see Mirecka et al. 2014).
Figure 2: Circular dichroism (CD) spectroscopy spectra of sequestrins, before (black) and after (grey) heat-induced denaturation and refolding. Spectra for Sq_{aSN2}, Sq_{aSN3} and Sq_{aSN4} are shown in Figure 2a. Spectra for Sq_{aSN6} and Sq_{aSN11} are shown in Figure 2b.
Figure 3: Surface plasmon resonance (SPR) sensorgram for the sequestrins Sq_{aSN2}, Sq_{aSN3}, Sq_{aSN4}, Sq_{aSN6}, and Sq_{aSN11} at **(A-C, G-I)** 25 °C and **(D-F, J-L)** 37 °C, respectively. The y-axis shows the relative response units (RU) and x-axis time (s). The black lines represent the fitting of the data to calculate kinetics. The experiment was conducted with immobilized aSN at a coating density of 190 RU.
Figure 4: Circular dichroism (CD) spectra between 195-260 nm for proteins in equimolar concentrations. **(A-C)** Indicates the structural rearrangement upon co-incubation of aSN and sequestrins. The spectra show the signal lost upon co-incubation between aSN and respective sequestrin. **(D-F)** Variable temperature measurements at 221 nm of sequestrins with and without aSN co-incubated with the sequestrins. **(G-I)** Sequestrins in complex with aSN before (light grey) and after (black) thermal melting.
Figure 5: Aggregation assay measuring thioflavin T (ThT) fluorescence at 480 nm. ThT fluorescence monitored for 75 h for 70 µM of respective aSN variants, and 1:1, 1:5 and 1:10 molar ratios of respective sequestrins. Standard deviation of the replicates is shown in the graph. See Figure 5a for Sq_{aSN2} and Sq_{aSN3}, Figure 5b for Sq_{aSN4} and Sq_{aSN6} and Figure 5c for Sq_{aSN11} and AS69 (reference, see Mirecka et al. 2014).

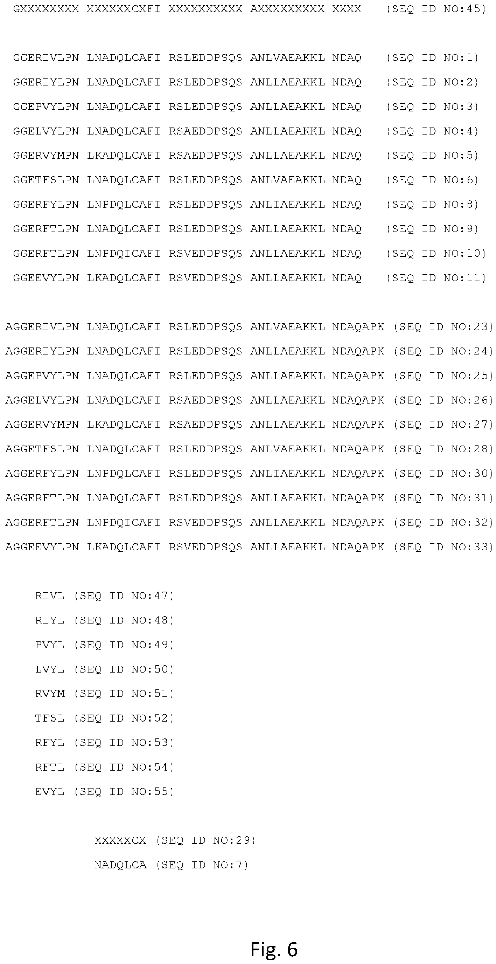

Figure 6: Alignment of sequences related to the first subunit.

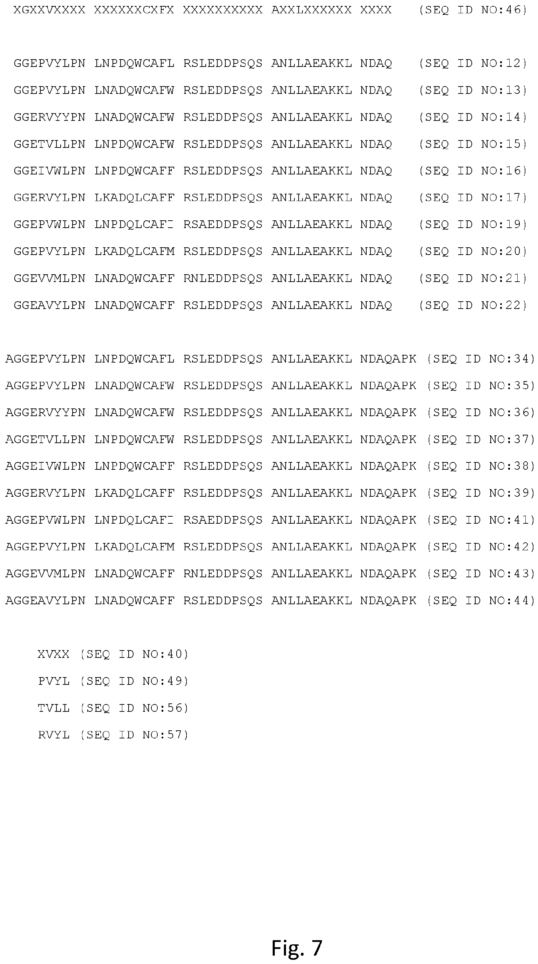

Figure 7: Alignment of sequences related to the second subunit.

### DETAILED DESCRIPTION

As a first aspect of the present disclosure, there is provided an alpha synuclein-binding polypeptide comprising a first and a second subunit and a linker linking the C-terminus of the first subunit to the N-terminus of the second subunit.

The first subunit comprises the amino acid sequence 
GX3X4X5X6X7X8X9X10X11X12X13X14X15X16CX18FIX21X22X23X24X25X26X27X28X29X30AX32
X33X34X35X36X37X38X39X40X41X42X43X44 (SEQ ID NO: 45),
 wherein
X₃ is G, R, P or K, preferably G or R, more preferably G,
X₄ is E, D, N or Q, preferably E,
X₅ is any amino acid except C, preferably R, P, L, T or E,
X₆ is any amino acid except C, preferably I, V or F,
X₇ is any amino acid except C, preferably Y, V, S or T,
X₈ is L or M, preferably L,
X₉ is P, T, R, L, Y, G or H, preferably P,
X₁₀ is N, E, D or Q, preferably N,
X₁₁ is L, A, I, V or G, preferably L,
X₁₂ is N or K, preferably N,
X₁₃ is A or P, preferably A,
X₁₄ is D, H, S, A or E, preferably D,
X₁₅ is Q, D, N or E, preferably Q,
X₁₆ is L or I, preferably L,
X₁₈ is A, I, V, G or L, preferably A or L, more preferably A,
X₂₁ is R, Q, N, H or K, preferably R,
X₂₂ is S, T, I, L or V, preferably S,
X₂₃ is L, A or V, preferably A,
X₂₄ is E, Q, D or N, preferably E or Q, more preferably E,
X₂₅ is D or E, preferably D,
X₂₆ is D or E, preferably D,
X₂₇ is P, R, Q, E, S or G, preferably P,
X₂₈ is S or T, preferably S,
X₂₉ is Q or N, preferably Q,
X₃₀ is S, Q, E, N or P, preferably S,
X₃₂ is N or Q, preferably N,
X₃₃ is L or I, preferably L,
X₃₄ is L or V, preferably L,
X₃₅ is A, I or L, preferably A,
X₃₆ is E or D, preferably E,
X₃₇ is A, I or L, preferably A,
X₃₈ is K or R, preferably K,
X₃₉ is K or R, preferably K,
X₄₀ is L, A, I or V, preferably L,
X₄₁ is N or Q, preferably N,
X₄₂ is D or E, preferably D,
X₄₃ is A, I or L, preferably A and
X₄₄ is Q or N, preferably Q.

The alternatives listed for X₅₋₈, X₁₂₋₁₃, X₁₆, X₂₃ and X₃₄ are based on the results presented in the Examples section below.

The alternatives listed for X₃₋₄, X₉₋₁₁, X₁₄₋₁₅, X₁₈, X₂₁₋₂₂, X₂₄₋₃₀, X₃₂₋₃₃ and X₃₅₋₄₄ are based on the inventors' extensive experience of working with the general sequestrin structures.

In one embodiment, the subsequence X₅X₆X₇X₈ is RIVL (SEQ ID NO:47), RIYL (SEQ ID NO:48), PVYL (SEQ ID NO:49), LVYL (SEQ ID NO:50), RVYM (SEQ ID NO:51), TFSL (SEQ ID NO:52), RFYL (SEQ ID NO:53), RFTL (SEQ ID NO:54) or EVYL (SEQ ID NO:55), preferably RIYL, PVYL, LVYL, TFSL or EVYL, more preferably RIYL, LVYL or TFSL.

The subsequence X₁₂X₁₃X₁₄X₁₅X₁₆CX₁₈ (SEQ ID NO:29) is preferably NADQLCA (SEQ ID NO:7).

In a particularly preferred embodiment, X₅X₆X₇X₈ is LVYL, X₁₂X₁₃ is NA, X₁₆ is L, X₂₃ is A and X₃₄ is L. These amino acids are found in Sq_{aSN4}, which is particularly advantageous according to table 4 below.

In one embodiment, first subunit comprises or consists of:
(i) a sequence selected from the groups consisting of SEQ ID NO:1, SEQ ID NO:2, SEQ ID NO:3, SEQ ID NO:4, SEQ ID NO:5, SEQ ID NO:6, SEQ ID NO:8, SEQ ID NO:9, SEQ ID NO:10 and SEQ ID NO:11; or
(ii) a sequence having at least 90%, such as at least 95%, identity to any one of SEQ ID NO:1, SEQ ID NO:2, SEQ ID NO:3, SEQ ID NO:4, SEQ ID NO:5, SEQ ID NO:6, SEQ ID NO:8, SEQ ID NO:9, SEQ ID NO:10 and SEQ ID NO:11.

The sequence of (i) is preferably SEQ ID NO:2, SEQ ID NO:3, SEQ ID NO:4 or SEQ ID NO:6, more preferably SEQ ID NO:2 or SEQ ID NO:4. These preferences also apply to (ii).

In another embodiment, the first subunit comprises or consists of:
(v) a sequence selected from SEQ ID NO:23, SEQ ID NO:24, SEQ ID NO:25, SEQ ID NO:26, SEQ ID NO:27, SEQ ID NO:28, SEQ ID NO:30, SEQ ID NO:31, SEQ ID NO:32 and SEQ ID NO:33; or
(vi) a sequence having at least 90%, such as at least 95%, identity to any one of SEQ ID NO:23, SEQ ID NO:24, SEQ ID NO:25, SEQ ID NO:26, SEQ ID NO:27, SEQ ID NO:28, SEQ ID NO:30, SEQ ID NO:31, SEQ ID NO:32 and SEQ ID NO:33.

The sequence of (v) is preferably SEQ ID NO:24, SEQ ID NO:25, SEQ ID NO:26 or SEQ ID NO:28, more preferably SEQ ID NO:24 or SEQ ID NO:26. These preferences also apply to (vi).

The term "% identity", as used throughout the specification, is calculated as follows. The query sequence is aligned to the target sequence using the CLUSTAL W algorithm (Thompson et al (1994) Nucleic Acids Research, 22:4673-4680). A comparison is made over the window corresponding to the target sequence. The amino acid residues at each position are compared and the percentage of positions in the query sequence that have identical correspondences in the target sequence is reported as % identity.

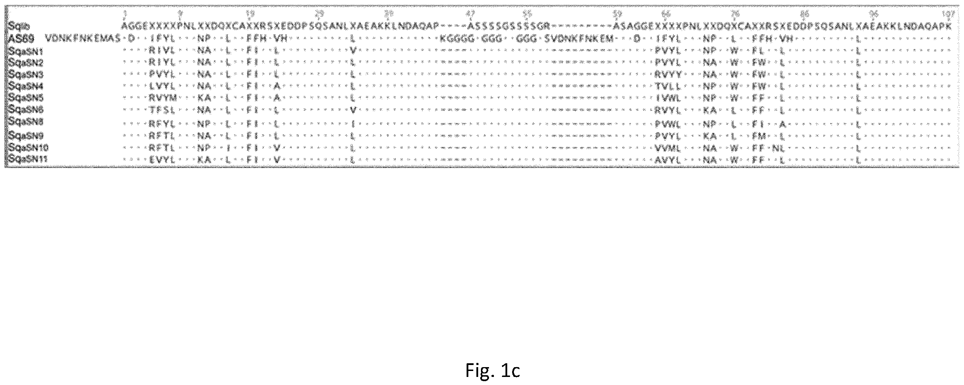

SEQ ID NO:23 is the first subunit of Sq_{aSN1} shown in figure 1c and discussed below. SEQ ID NO:1 is a subsequence of SEQ ID NO:23.

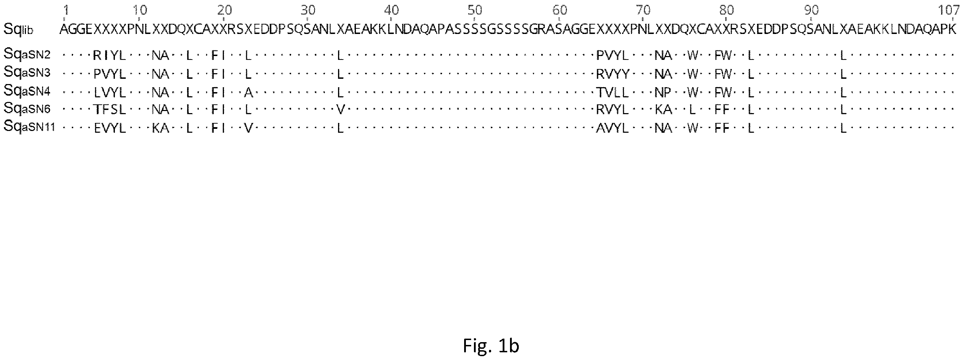

SEQ ID NO:24 is the first subunit of Sq_{aSN2} shown in figure 1b-c and discussed below. SEQ ID NO:2 is a subsequence of SEQ ID NO:24.

SEQ ID NO:25 is the first subunit of Sq_{aSN3} shown in figure 1b-c and discussed below. SEQ ID NO:3 is a subsequence of SEQ ID NO:25.

SEQ ID NO:26 is the first subunit of Sq_{aSN4} shown in figure 1b-c and discussed below. SEQ ID NO:4 is a subsequence of SEQ ID NO:26.

SEQ ID NO:27 is the first subunit of Sq_{aSN5} shown in figure 1b and discussed below. SEQ ID NO:5 is a subsequence of SEQ ID NO:27.

SEQ ID NO:28 is the first subunit of Sq_{aSN6} shown in figure 1b-c and discussed below. SEQ ID NO:6 is a subsequence of SEQ ID NO:28.

SEQ ID NO:30 is the first subunit of Sq_{aSN8} shown in figure 1b and discussed below. SEQ ID NO:8 is a subsequence of SEQ ID NO:30.

SEQ ID NO:31 is the first subunit of Sq_{aSN9} shown in figure 1b and discussed below. SEQ ID NO:9 is a subsequence of SEQ ID NO:31.

SEQ ID NO:32 is the first subunit of Sq_{aSN10} shown in figure 1b and discussed below. SEQ ID NO:10 is a subsequence of SEQ ID NO:32.

SEQ ID NO:33 is the first subunit of Sq_{aSN11} shown in figure 1b-c and discussed below. SEQ ID NO:11 is a subsequence of SEQ ID NO:33.

The second subunit comprises the amino acid sequence 
X62GX64X65VX67X68X69X70X71X72X73X74X75X76CX78FX80X81X82X83X84X85X86X87X88X89
X90AX92X93LX95X96X97X98X99X100X101X102X103X104 (SEQ ID NO:46),
 wherein
X₆₂ is G or P, preferably G,
X₆₄ is E or D, preferably E,
X₆₅ is any amino acid except C, such as P, R, T, I, V or A, preferably P, R or T,
X₆₇ is any amino acid except C, preferably Y, L, W or M, more preferably Y or L,
X₆₈ is L or M, preferably L,
X₆₉ is P, L, T, R, L, Y or H, preferably P,
X₇₀ is N, E, D or Q, preferably N,
X₇₁ is L, A, I, V or G, preferably L,
X₇₂ is N or K, preferably N,
X₇₃ is A or P, preferably P,
X₇₄ is D, H, S or A, preferably D,
X₇₅ is Q or N, preferably Q,
X₇₆ is W or L, preferably W,
X₇₈ is A, I, V, G or L, preferably A,
X₈₀ is L, W, F, I or M, preferably L, W, For M, more preferably W or F, most preferably W,
X₈₁ is R, Q, N, H or K, preferably R,
X₈₂ is S, T, I, L or V, preferably S,
X₈₃ is L or A, preferably L,
X₈₄ is E, Q, D or N, preferably E,
X₈₅ is D or E, preferably D,
X₈₆ is D or E, preferably D,
X₈₇ is P, R, Q, E, S or G, preferably P,
X₈₈ is S or T, preferably S,
X₈₉ is Q or N, preferably Q,
X₉₀ is S, Q, E, N or P, preferably S,
X₉₂ is N or Q, preferably N,
X₉₃ is L or I, preferably L,
X₉₅ is A, I or L, preferably A,
X₉₆ is E or D, preferably E,
X₉₇ is A, I or L, preferably A,
X₉₈ is K or R, preferably K,
X₉₉ is K or R, preferably K,
X₁₀₀ is L, A, I or V, preferably L,
X₁₀₁ is N or Q, preferably N,
X₁₀₂ is D or E, preferably D,
X₁₀₃ is A, I or L, preferably A, and
X₁₀₄ is Q or N, preferably Q.

The alternatives listed for X₆₅, X₆₇₋₆₈, X₇₂₋₇₃, X₇₆, X₈₀ and X₈₃ are based on the results presented in the Examples section below.

The alternatives listed for X₆₂, X₆₄, X₆₉₋₇₁, X₇₄₋₇₅, X₇₈, X₈₁₋₈₂, X₈₄₋₉₀, X₉₂₋₉₃ and X₉₅₋₁₀₄ are based on the inventors' extensive experience of working with the general sequestrin structures.

In one embodiment, the subsequence X₆₅VX₆₇X₆₈ (SEQ ID NO:40) is PVYL, TVLL (SEQ ID NO:56) or RVYL (SEQ ID NO:57), preferably PVYL or TVLL.

In another embodiment, X₇₆ and X₈₀ are both W.

In a particularly preferred embodiment, X₆₅VX₆₇X₆₈ is TVLL, X₇₂X₇₃ is NP, X₇₆ and Xso are both W and X₈₃ is L. These amino acids are found in Sq_{aSN4}, which is particularly advantageous according to table 4 below.

In one embodiment, the second subunit comprises or consists of:
(iii) a sequence selected from SEQ ID NO:12, SEQ ID NO:13, SEQ ID NO:14, SEQ ID NO:15, SEQ ID NO:16, SEQ ID NO:17, SEQ ID NO:19, SEQ ID NO:20, SEQ ID NO:21 and SEQ ID NO:22; or
(iv) a sequence having at least 90%, such as at least 95%, identity to any one of SEQ ID NO:12, SEQ ID NO:13, SEQ ID NO:14, SEQ ID NO:15, SEQ ID NO:16, SEQ ID NO:17, SEQ ID NO:19, SEQ ID NO:20, SEQ ID NO:21 and SEQ ID NO:22.

The sequence of (iii) is preferably SEQ ID NO:13, SEQ ID NO:14, SEQ ID NO:15 or SEQ ID NO:17, more preferably from SEQ ID NO:13 or SEQ ID NO:15. The same preferences apply to (iv).

In another embodiment, the second subunit comprises or consists of:
(vii) a sequence selected from SEQ ID NO:34, SEQ ID NO:35, SEQ ID NO:36, SEQ ID NO:37, SEQ ID NO:38, SEQ ID NO:39, SEQ ID NO:41, SEQ ID NO:42, SEQ ID NO:43 and SEQ ID NO:44; or
(viii) a sequence having at least 90%, such as at least 95%, identity to any one of SEQ ID NO:34, SEQ ID NO:35, SEQ ID NO:36, SEQ ID NO:37, SEQ ID NO:38, SEQ ID NO:39, SEQ ID NO:41, SEQ ID NO:42, SEQ ID NO:43 and SEQ ID NO:44.

The sequence of (vii) is preferably SEQ ID NO:35, SEQ ID NO:36, SEQ ID NO:37 or SEQ ID NO:39, more preferably SEQ ID NO:35 or SEQ ID NO:37. The same preferences apply to (viii).

SEQ ID NO:34 is the second subunit of Sq_{aSN1} shown in figure 1c and discussed below. SEQ ID NO:12 is a subsequence of SEQ ID NO:34.

SEQ ID NO:35 is the second subunit of Sq_{aSN2} shown in figure 1b-c and discussed below. SEQ ID NO:13 is a subsequence of SEQ ID NO:35.

SEQ ID NO:36 is the second subunit of Sq_{aSN3} shown in figure 1b-c and discussed below. SEQ ID NO:14 is a subsequence of SEQ ID NO:36.

SEQ ID NO:37 is the second subunit of Sq_{aSN4} shown in figure 1b-c and discussed below. SEQ ID NO:15 is a subsequence of SEQ ID NO:37.

SEQ ID NO:38 is the second subunit of Sq_{aSN5} shown in figure 1b and discussed below. SEQ ID NO:16 is a subsequence of SEQ ID NO:38.

SEQ ID NO:39 is the second subunit of Sq_{aSN6} shown in figure 1b-c and discussed below. SEQ ID NO:17 is a subsequence of SEQ ID NO:39.

SEQ ID NO:41 is the second subunit of Sq_{aSN8} shown in figure 1b and discussed below. SEQ ID NO:19 is a subsequence of SEQ ID NO:41.

SEQ ID NO:42 is the second subunit of Sq_{aSN9} shown in figure 1b and discussed below. SEQ ID NO:20 is a subsequence of SEQ ID NO:42.

SEQ ID NO:43 is the second subunit of Sq_{aSN10} shown in figure 1b and discussed below. SEQ ID NO:21 is a subsequence of SEQ ID NO:43.

SEQ ID NO:44 is the second subunit of Sq_{aSN11} shown in figure 1b-c and discussed below. SEQ ID NO:22 is a subsequence of SEQ ID NO:44.

The linker comprises at least eight amino acid residues, such as 8-30 amino acid residues, such as 8-25 amino acid residues, such as 10-25 amino acid residues.

The linker may for example comprise at least four S residues. In one embodiment, the linker comprises the sequence SSSSGSSSSG (SEQ ID NO:18).

As a second aspect of the present disclosure, there is provided a fusion protein comprising a polypeptide according to the first aspect and a half-life-extending region.

The half-life-extending region may for example be an Fc-binding region or an albumin-binding region (ABR). The half-life-extending region may for example be located on the C-terminal side of the alpha synuclein-binding polypeptide.

Alternatively, a half-life-extending group is connected to the the alpha synuclein-binding polypeptide in another way. In this alternative embodiment, the polypeptide and the half-life-extending group together forms a construct that may comprise further parts or groups.

Various half-life-extending strategies for polypeptides/proteins are described in a review article by Kontermann (EXPERT OPINION ON BIOLOGICAL THERAPY, 2016 VOL. 16, NO. 7, 903-915).

As a third aspect of the present disclosure, there is provided a fusion protein comprising a polypeptide according to the first aspect and a region facilitating transport across the blood-brain barrier. Such a region may for example bind to transferrin receptor 1.

Alternatively, a group facilitating transport across the blood-brain barrier is connected to the alpha synuclein-binding polypeptide in another way. In this alternative embodiment, the polypeptide and the transport-facilitating group together forms a construct that may comprise further parts or groups.

In one embodiment, the fusion protein of the second aspect comprises the transport-facilitating group of the third aspect.

As a fourth aspect of the present disclosure, there is provided a polypeptide according to the first aspect or a fusion protein according to the second or third aspect for use as a medicament.

As a fifth aspect of the present disclosure, there is provided a pharmaceutical composition comprising the polypeptide or fusion protein according to any one of the preceding aspects. The composition of the fifth aspect is preferably adapted for intravenous or subcutaneous injection.

As a sixth aspect of the present disclosure, there is provided a polypeptide, fusion protein or pharmaceutical composition according to any one of the preceding aspects for use in a method of preventing or treating Parkinson's disease or Lewy body dementia.

As a seventh aspect of the present disclosure, there is provided a method of preventing or treating Parkinson's disease or Lewy body dementia in a subject in need thereof, said method comprising administration of a polypeptide, fusion protein or pharmaceutical composition according to any one of the preceding aspects.

The method of the sixth or seventh aspect typically comprises injection, such as intravenous or subcutaneous injection, of the polypeptide, fusion protein or pharmaceutical composition.

The subject of the method of the sixth or seventh aspect is preferably human.

### EXAMPLES

### Methods

### Phage display selections against alpha synuclein (1-140)

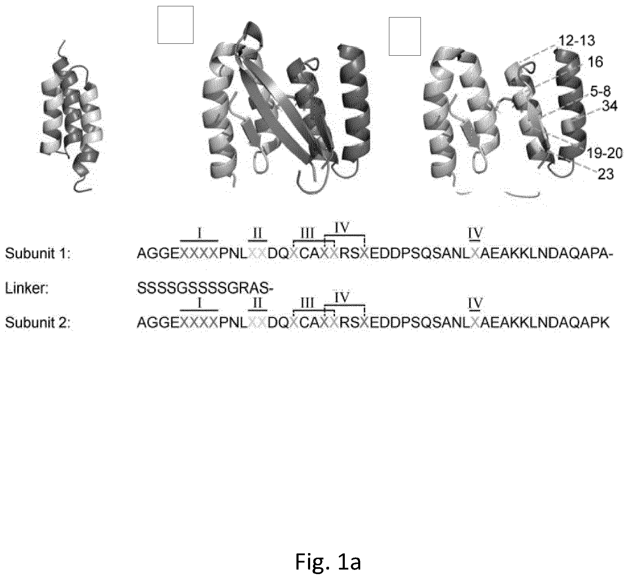

Phage display selection from the Sq_{lib} (Hjelm et al. 2023) was performed using biotinylated alpha synuclein (aSN) 1-140 as target (AnaSpec, United States) in five rounds (the randomization behind the library is further explained in figure 1a). The target concentration was decreased over the five selection rounds (200 nM, 200 nM, 100 nM, 50 nM, 25 nM) and performed at 4 °C in round one to four. In the fifth round, the selection was divided into two tracks with one at 4 °C and one at room temperature. The library was incubated with target for around 16 hours in the first round, and for 1 hour in rounds 2-5. The stringency of the selection was increased with each round by increasing the number and time of washing steps with PBSTB (5×1 min, 5×2 min, 5×3min, 5×6 min, and 5×8 min).

### Phage ELISA

To investigate the efficacy of the selection, polyclonal ELISA from phage stock after each selection round and monoclonal phage ELISA on individual clones were performed as described previously (Hjelm et al. 2023). All phage stocks were diluted to the same concentration before incubating with aSN (1-140).

The ELISA plate was prepared in a 384-well format (Nunc, PS, Low binding, Hi-Edge, clear), with four wells per individual clone. Two wells per clone were coated with HSA [5 µg/ml] or BSA [1 w/v%]. Two wells where precoated with streptavidin [5 µg/ml] and coated in a second step with biotinylated aSN [1 µg/ml] or BSA [1 w/v%]. Signal corresponding to binding to aSN was normalized to the HSA signal and blanked by subtraction from BSA or streptavidin-BSA background signal. DNA sequences were identified with Sanger sequencing (Microsynth SeqLab, Germany), and sequences analysed with the Geneious software (version 11.2, Biomatters LTD, New Zealand).

### Expression and purification of soluble sequestrins

DNA encoding the sequestrins were amplified from phagemids with primers designed for the In-Fusion HD cloning kit (Takara Bio Europe), by manufacturer's recommendations into the pET-26b(+)-vector for periplasmic production with a C-terminal His₆-tag. Sequence verified clones (Sanger sequencing, Eurofins Genomics, Germany) were heat-shocked to the *E. coli* strain BL21(DE3) for protein expression. Cultivation was started from over-night inoculated colonies to an OD₆₀₀ of 0.1 AU in TSBY with kanamycin [25 µg/ml] and grown until OD₆₀₀ reached 1.0 AU at 37°C before inducing with 1 mM IPTG and thereafter incubated at 25 °C for 16 hours. Cell pellets from harvest were dissolved in IMAC running buffer [47 mM Na₂HPO₄, 3 mM NaH₂PO₄, 300 mM NaCl, 15 mM imidazole, pH 7.4] before proceeding with purification.

The harvested cells were sonicated using a Vibra-Cell VCX 130 sonicator (Sonics, United States) and cell debris removed by centrifugation and filtration. The lysate was loaded on an equilibrated HisPur Cobalt Resin (Thermo Scientific, United States) and washed with running buffer. The sample was isocraticly eluted with the running buffer supplemented with 150 mM imidazole. Fractions of eluate containing protein according to measurement with the Pierce^{™} BCA Protein Assay Kit (Thermo Scientific, United States) were pooled and buffer exchanged to PBS on PD 10 desalting columns (Cytiva, United States). The eluted samples were loaded (1.5 µg) on a gel for SDS-PAGE analysis (NuPAGE Bis-Tris 4-12%, Invitrogen, United States). Molecular mass was determined by a Thermo Ultimate3000 Bruker Impact II system connected to a ProSwift RP-4H, 1×50 mm column (Thermo Fisher, United States) using a linear gradient elution with acetonitrile [3% to 95%], supplemented with 0.1% formic acid.

### Circular dichroism spectroscopy

The secondary structure of the sequestrins was analysed using the Chirascan system (Applied Photophysics, United Kingdom) with a 1 mm High precision cell (110-1P-40 cuvettes, Hellma Analytics, Germany). Five wavelength scans were recorded and averaged between 195 nm and 260 nm at 20 °C on a protein sample of 0.2 mg/ml in PBS.

The melting point was determined by using a temperature gradient of 1 °C per minute at 221 nm for five average readings at each sample point. The refolding capability was assessed by repeating the spectral scan after the sample had been subjected to heat treatment and cooled down to 20 °C. The spectra from before and after thermal heating was compared to assess the refolding capability of each protein.

For analyzing the secondary structure during interaction with aSN, equimolar concentrations of 15 µM of the sequestrin and aSN was co-incubated and analyzed by circular dichroism spectroscopy. Secondary structure content was approximated by BeStSel algorithm (Micsonai et al. 2015).

### Biosensor analysis

Sequestrins were analysed on a Biacore 8K system (Cytiva, United States) with a Series S SA sensor chip (Cytiva, United States) immobilized with biotinylated aSN 1-140 (ALN-H82H8, ACRO Biosciences, United States) to 190 RU in PBST as running buffer (0.05% Tween20). Samples were injected for 350 seconds and the dissociation was recorded for 1,500 seconds at 30 µl/min, before regeneration with 10 mM HCL for 35 seconds at 30 µl/min. Samples were analysed in a dilution series in duplicate at 25 °C and 37 °C, respectively. The results were evaluated using the Multi-cycle kinetics method - 1:1 binding and 1:1 dissociation, by the Biacore Insight Evaluation Software (Version 2.0.15, Cytiva, United States).

### Preparation of the recombinant aSN monomers

Recombinant human wt and mutated aSN were expressed in *E. coli* and purified as described before (Paslawski et al. 2019; Paslawski, Lorenzen, and Otzen 2016) Briefly, aSN plasmid vector pET11-D, containing the insert coding human aSN, was expressed in *E*. *coli* BL21 (DE3) competent cells using an auto-induction method. Cells were harvested by centrifugation and treated with the osmotic shock buffer (20 mM Tris-HCl, pH: 7.2, 40% sucrose), incubated for 10 min and centrifuged again. Afterwards, the pellet was suspended in ice-cold deionised water, with the subsequent addition of saturated MgCl₂, and briefly incubated on ice. The periplasmic fraction of the cell lysate was collected, and the majority of unwanted proteins were precipitated by acidification. The solution was fractionated on a Q-Sepharose column connected to an ÄKTA Explorer system (Cytiva, United States). Fractions containing aSN were identified by SDS-PAGE, pulled together and high molecular weight aggregates were removed by filtration through a 30 kDa filter. The aSN concentration was determined using NanoDrop ND1000 (Thermo Fisher Scientific, United States), and the protein was aliquoted, lyophilized and stored at -20 °C.

### Alpha synuclein aggregation

The sequestrins from IMAC purification were additionally purified by size exclusion chromatography (SEC). Samples were added to a HiLoad 16/600 200 pg (Cytiva, Marlborough, MA, USA) column with PBS as running buffer on a ÄKTA PURE system (Cytiva, Marlborough, MA, USA). The purified proteins were analyzed again by PierceTM BCA Protein Assay Kit (Thermo Scientific, Waltham, MA, USA), SDS-PAGE and mass spectrometry as described above. For aSN aggregation, recombinant human wt and mutated aSN, respectively, were incubated with or without the addition of sequestrins, at a final concentration of 70 µM for aSN and respectively 70, 14 and 7 µM for sequestrins, with 40 µM ThT in a FLUOstar Omega (BMG Labtech, Ortenberg, Germany) plate reader at 37 °C with shaking. The ThT signal was monitored at 448 nm excitation and 482 nm emission.

### Results

### Phage display selection of sequestrins against alpha synuclein

For selection of new sequestrins against alpha synuclein (aSN), a previously described (Hjelm et al. 2023) naive sequestrin library (Sq_{lib}) displayed on phage was used in five biopanning cycles with decreasing amount of target peptide and increased stringency. The first four cycles were carried out at 4 °C to minimize aggregation of aSN during selection. In the fifth cycle, the phage pool was split in two, with one track at 4°C and the other at room temperature. The polyclonal ELISA showed an increase in target-binding signal from the first to the third cycle and a relatively constant signal after the third cycle. The results from the ELISA correlated with the observed enrichment of phage titres for the third selection cycle. The monoclonal ELISA on isolated variants from the later cycles showed a relatively high proportion of positive clones for target antigen, increasing from around 33% in cycle four to 60% in cycle five. At the sequence level, the proportion of amino acids in randomised positions, compared to the original library, shifted for several positions where some amino acids were dominant. An average of six substitutions per protein, ranging between four to nine substitutions, was observed. Representative clones from different clusters in the phylogenetic tree were selected for further characterization (figure 1b-c).

### Recombinant production of sequestrins targeting alpha synuclein

The five clones showing the highest affinity were selected, subcloned to an expression vector in fusion to a C-terminal his-tag (Sq_{aSN}-His₆), produced in *E. coli* and purified using immobilized metal ion affinity chromatography (table 1). One previously described binder, with specificity for aSN (AS69), was included as control (Mirecka et al. 2014). The molecular weight of the produced proteins was confirmed by mass spectrometry and had an experimental mass similar to the expected mass (table 1).

**Table 1. Production yield for Sq_{aSN} clones and molecular weight (Mw) as determined by LC_MS (ESI).**

| Sq_{aSN} clone | Yield protein per 100 ml culture [mg] | Mw_{expected} [Da] | Mw_{observed} [Da] |
|---|---|---|---|
| Sq_{aSN2}-His6 | 4.0 | 12,702 | 12,700 |
| Sq_{aSN3}-His6 | 4.7 | 12,735 | 12,736 |
| Sq_{aSN4}-His6 | 4.2 | 12,580 | 12,581 |
| Sq_{aSN6}-His6 | 2.0 | 12,552 | 12,550 |
| Sq_{aSN11}-His6 | 5.4 | 12,596 | 12,594 |

### Secondary structure and thermal stability of sequestrins

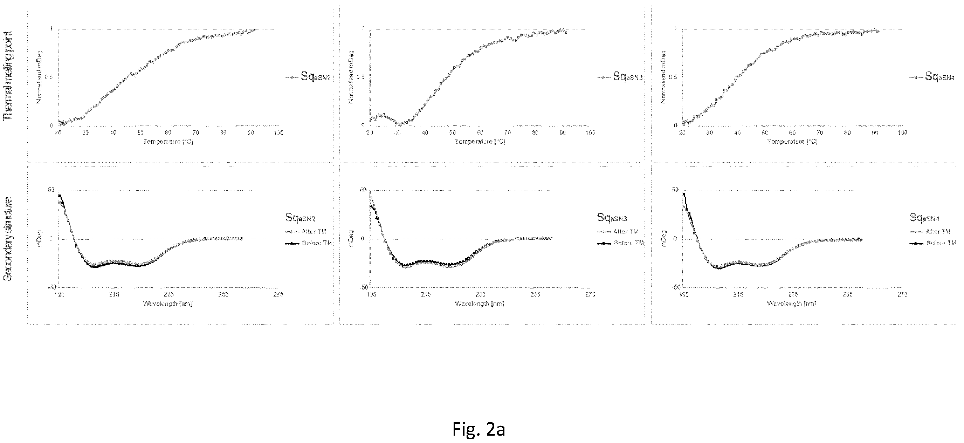

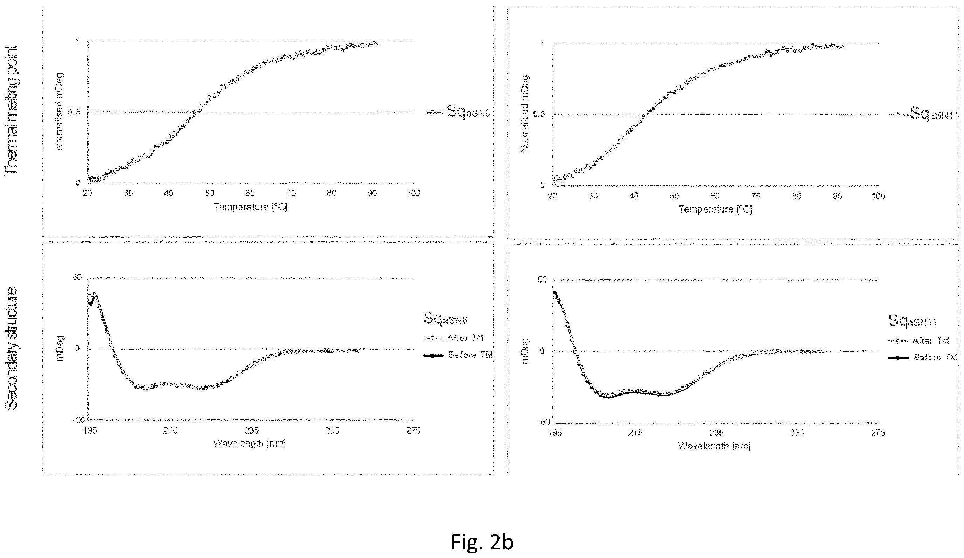

The secondary structure content, thermal stability and refolding of the selected sequestrins was analyzed by circular dichroism (CD) spectroscopy. The sequestrins showed an alpha helical content that was similar to previously reported sequestrins targeting amyloid beta (Hjelm et al. 2023; Hoyer and Härd 2008) (figure 2). Thermal melting point (Tₘ) was assessed by variable temperature measurement (VTM) and the average Tₘ was around 44 °C and ranging between 38-48 °C (table 2). The CD spectra from before and after VTM showed good overlap, indicating refolding after heat-induced denaturation (figure 2).

**Table 2. Kinetic rate constants (kₐ and k_{d}) and affinity (K_{D}) for immobilized aSN as determined by SPR against sequestrin clones as analytes at 25 °C. Melting temperature (Tₘ) and refolding as determined by circular dichroism spectroscopy.**

| Sq_{aSN} clone | kₐ [1/Ms] | k_{d} [1/s] | K_{D} [nM] | Tₘ [°C] | Refolding |
|---|---|---|---|---|---|
| AS6₉ | 1.58 × 10³ | 1.14 × 10⁻⁴ | 72.2 | n.d. | n.d. |
| Sq_{aSN2} | 6.37 × 10³ | 6.43 × 10⁻⁵ | 10.1 | 44 | Yes |
| Sq_{aSN3} | 7.60 × 10³ | 7.98 × 10⁻⁵ | 10.5 | 48 | Yes |
| Sq_{aSN4} | 9.41 × 10³ | 8.49 × 10⁻⁵ | 9.02 | 40 | Yes |
| Sq_{aSN6} | 5.26 × 10³ | 9.45 × 10⁻⁵ | 17.9 | 47 | Yes |
| Sq_{aSN11} | 6.81 × 10³ | 9.61 × 10⁻⁵ | 14.1 | 42 | Yes |

### Biosensor analysis of selected sequestrins targeting alpha synuclein

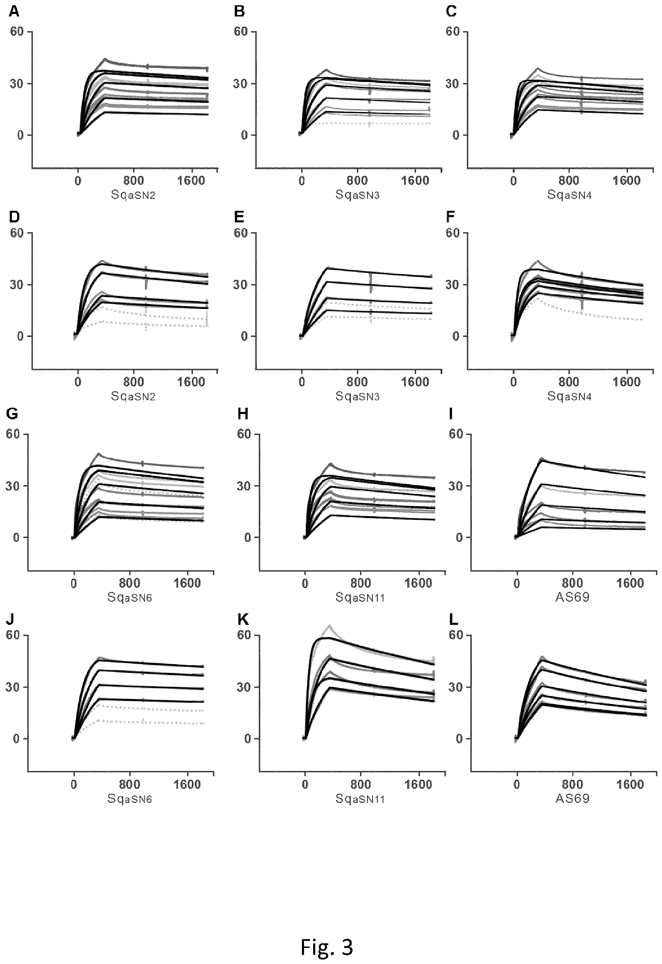

Surface plasmon resonance was used to assess the kinetics of the interactions between the selected sequestrins and aSN. Biotinylated aSN (bio-aSN) was captured onto streptavidin-coated chips, followed by injection of the sequestrins. The kinetics for the interactions demonstrated a general trend of relatively slow association and slow dissociation and the best variants had equilibrium dissociation constants (K_{D}) of around 10 nM (figure 3). Compared to the previously reported dimeric affibody binder for aSN (Mirecka et al. 2014), the affinities of the sequestrins Sq_{aSN2}, Sq_{aSN3}, Sq_{aSN4}, Sq_{aSN6} and Sq_{aSN11} were around 7-fold higher (table 2). The clones were also analyzed at 37 °C. At the higher temperature, the kinetics was somewhat faster with similar K_{D} (figure 3, table 3).

**Table 3. Kinetic rate constants (kₐ and k_{d}) and affinity (K_{D}) for immobilized aSN as determined by SPR against sequestrin clones as analytes at 37 °C.**

| Sq_{aSN} clone | kₐ [1/Ms] | k_{d} [1/s] | K_{D} [nM] |
|---|---|---|---|
| AS69 | 3.88 × 10³ | 2.52 × 10⁻⁴ | 64.9 |
| Sq_{aSN2} | 7.46 × 10³ | 1.32 × 10⁻⁴ | 17.7 |
| Sq_{aSN3} | 3.13 × 10³ | 9.04 × 10⁻⁵ | 28.9 |
| Sq_{aSN4} | 1.49 × 10⁴ | 1.88 × 10⁻⁴ | 12.6 |
| Sq_{aSN6} | 5.48 × 10³ | 5.66 × 10⁻⁵ | 10.3 |
| Sq_{aSN11} | 7.70 × 10³ | 2.02 × 10⁻⁴ | 26.2 |

### Sequestrins targeting aSN changes secondary structure upon target binding

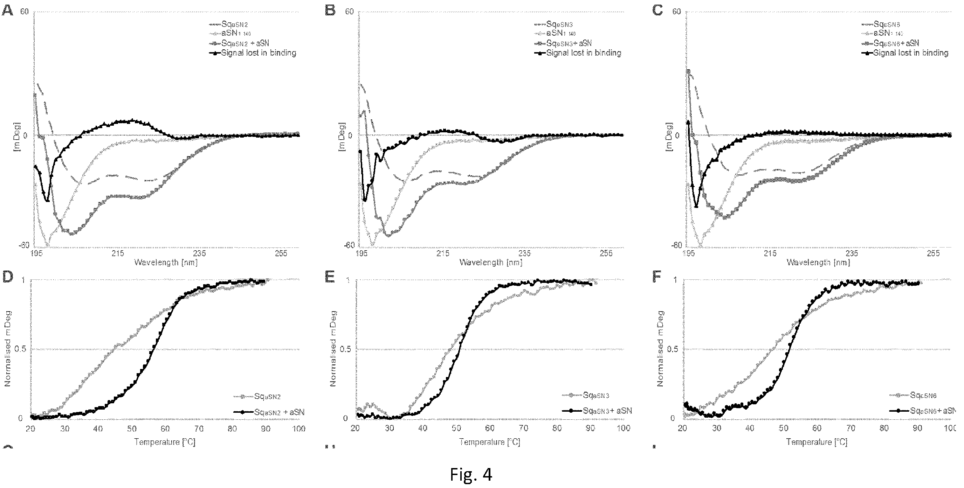

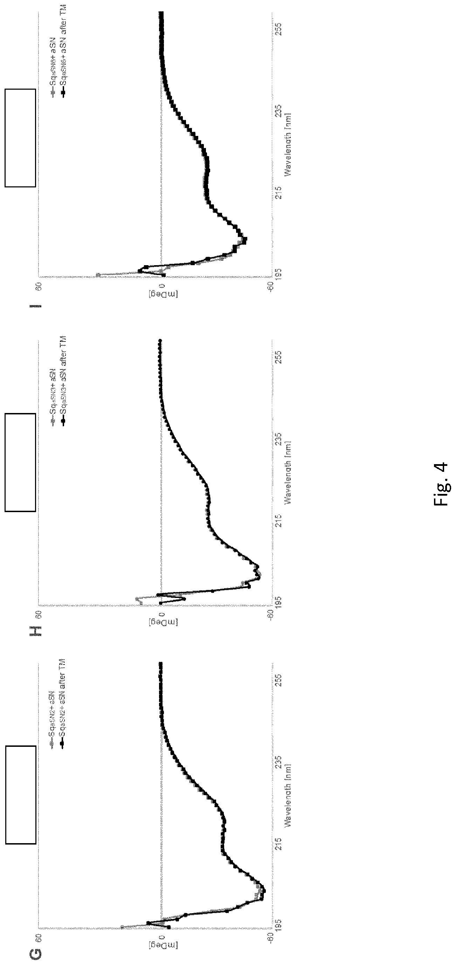

It has previously been shown that amyloid-beta binding sequestrins undergo structural rearrangements when binding the peptide (Hjelm et al. 2023; Hoyer and Härd 2008). CD spectroscopy on samples with the high affinity clonalities, Sq_{aSN2}, Sq_{aSN3}, and Sq_{aSN6} and co-incubated with aSN, respectively, was used to detect changes in secondary structure content. First, CD spectra of free sequestrins and aSN were recorded, showing alpha helical content for the sequestrins and random coil for aSN (figure 4A-C). When co-incubating aSN with respective sequestrin a loss of signal, mainly corresponding to random coil, is observed (figure 4A-C), indicating structural rearrangements in secondary structure upon binding. Moreover, co-incubation resulted in an increase in melting temperature (Tₘ), showing that the formed complex is stabilizing the overall structure (figure 4D-I).

### Alpha synuclein aggregation

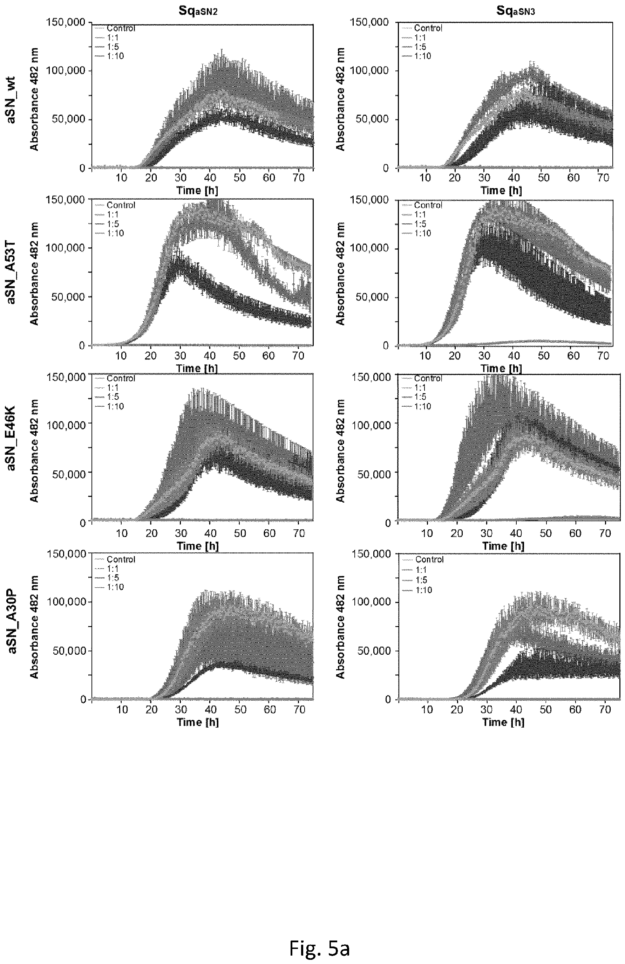

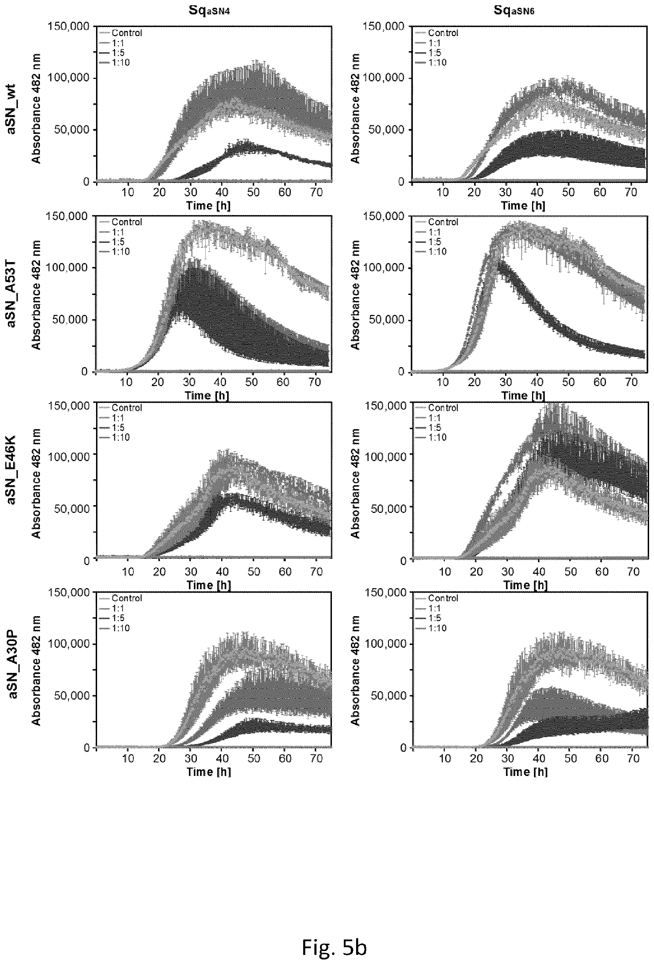

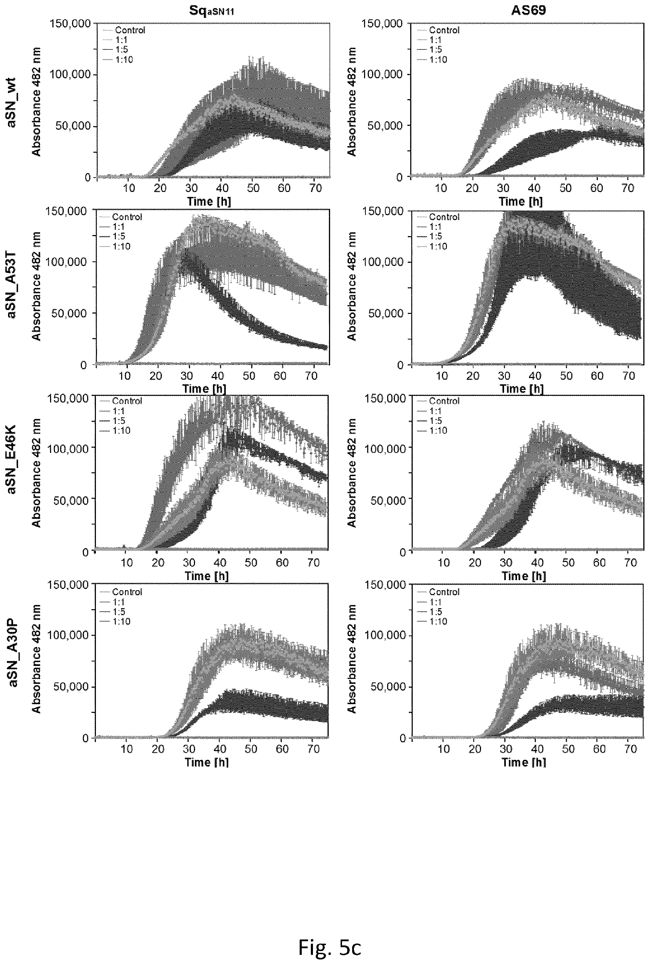

The effect of sequestrin-binding on aSN aggregation was analyzed using thioflavin T (ThT) fluorescence. Human wild type and three different familial aSN variants (A30P, E46K and A53T) were included in the assay, as well as the five selected sequestrins (Sq_{aSN2}, Sq_{aSN3}, Sq_{aSN4}, Sq_{aSN6}, and Sq_{aSN11}) and the previously described aSN-binder AS69 as control (Mirecka et al. 2014). Recombinant human wt and mutated aSN were expressed in *E. coli* and monomeric proteins were prepared using ion exchange chromatography and filtration. The aSN variants, respectively, were incubated with or without the addition of sequestrins, at a final concentration of 70 µM for aSN and respectively 70 (1:1), 14 (1:5) and 7 µM (1:10) for sequestrins, together with ThT in a plate reader at 37 °C with shaking, and the ThT fluorescence was monitored for 75 h. The samples with only aSN showed the expected timedependent increase in signal corresponding to aSN aggregation and the A53T mutant displayed a more rapid increase in signal in agreement with its higher aggregation propensity (Flagmeier et al. 2016) (figure 5). Co-incubation with equimolar concentration of respective sequestrin completely inhibited the aggregation of wt aSN and the three mutants (figure 5). More pronounced differences between the sequestrins were observed for the co-incubations with lower concentrations of binder in relation to aSN. Sq_{aSN4} showed for example an effect on the more aggregation-prone A53T variant of aSN, at both 1:5 and 1:10 molar ratio (figure 5).

Based on the results in figure 5, the binders were ranked. The complete ranking is shown in table 4 below.

**Table 4. Ranking of the binders in figure 5.**

| | Sq_{aSN2} | Sq_{aSN3} | Sq_{aSN4} | Sq_{aSN6} | Sq_{aSN11} | AS6₉ |
|---|---|---|---|---|---|---|
| aSN_wt | 4 | 5 | 1 | 2 | 6 | 3 |
| aSN_A53T | 2 | 5 | 1 | 4 | 3 | 6 |
| aSN_E46K | 2 | 3 | 1 | 6 | 5 | 4 |
| aSN_A30P | 4 | 3 | 2 | 1 | 6 | 5 |
| Total ranking score | 12 | 16 | 5 | 13 | 20 | 18 |

According to Table 4, Sq_{aSN4} is thus (clearly) the best binder followed by Sq_{aSN2}, Sq_{aSN6} and Sq_{aSN3}.

### REFERENCES

Breydo, Leonid, Jessica W. Wu, and Vladimir N. Uversky. 2012. "α-Synuclein Misfolding and Parkinson's Disease." Biochimica et Biophysica Acta - Molecular Basis of Disease 1822(2):261-85. doi: 10.1016/j.bbadis.2011.10.002
Flagmeier, Patrick, Georg Meisl, Michele Vendruscolo, Tuomas P. J. Knowles, Christopher M. Dobson, Alexander K. Buell, and Céline Galvagnion. 2016. "Mutations Associated with Familial Parkinson's Disease Alter the Initiation and Amplification Steps of α-Synuclein Aggregation." Proceedings of the National Academy of Sciences of the United States of America 113(37):10328-33. doi: 10-1073/pnas.1604645113.
Fornai, Francesco, Oliver M. Schlü ter, Paola Lenzi, Marco Gesi, Riccardo Ruffoli, Michela Ferrucci, Gloria Lazzeri, Carla L. Busceti, Fabrizio Pontarelli, Giuseppe Battaglia, Antonio Pellegrini, Ferdinando Nicoletti, Stefano Ruggieri, Antonio Paparelli, and Thomas C. Südhof. 2004. "Parkinson-like Syndrome Induced by Continuous MPTP Infusion: Convergent Roles of the Ubiquitin-Proteasome System and-Synuclein." PNAS 3413-18. doi: 10.1073/pnas.0409713102
Grönwall, Caroline, Andreas Jonsson, Sara Lindström, Elin Gunneriusson, Stefan Stahl, and Nina Herne. 2007. "Selection and Characterization of Affibody Ligands Binding to Alzheimer Amyloid β Peptides." Journal of Biotechnology 128(1):162-83. doi: 10.1016/j.jbiotec.2006.09.013.
Guan, Yuan, Xiaofang Zhao, Fengwei Liu, Shuxin Yan, Yalong Wang, Cuilian Du, Xiuyu Cui, Rena Li, and Claire Xi Zhang. 2020. "Pathogenic Mutations Differentially Regulate Cell-to-Cell Transmission of α-Synuclein." Frontiers in Cellular Neuroscience 14. doi: 10.3389/61061.2020.00159.
Hjelm, Linnea Charlotta, Hanna Lindberg, Stefan Stahl, and John Löfblom. 2023. "Construction and Validation of a New Naive Sequestrin Library for Directed Evolution of Binders against Aggregation-Prone Peptides." International Journal of Molecular Sciences 24(1):836. doi: 10.3390/ijms24010836.
Hoyer, Wolfgang, and Torleif Härd. 2008. "Interaction of Alzheimer's Aβ Peptide with an Engineered Binding Protein-Thermodynamics and Kinetics of Coupled Folding-Binding." Journal of Molecular Biology 378(2):398-411. doi: 10.10167j.jmb.2008.02.040.
Liu, W., J. Jalewa, M. Sharma, G. Li, L. Li, and C. Hölscher. 2015. "Neuroprotective Effects of Lixisenatide and Liraglutide in the 1-Methyl-4-Phenyl-1,2,3,6-Tetrahydropyridine Mouse Model of Parkinson's Disease." Neuroscience 303:42-50. doi: 10.1016/j.neuroscience.2015.06.054.
Löfblom, John, Fredrik Y. Frejd, and Stefan Stahl. 2011. "Non-Immunoglobulin Based Protein Scaffolds." Current Opinion in Biotechnology 22(6):843-48. doi: 10.1016/j.copbio.2011.06.002
Maiti, Panchanan, Jayeeta Manna, Gary L. Dunbar, Panchanan Maiti, and Gary L. Dunbar. 2017. "Current Understanding of the Molecular Mechanisms in Parkinson's Disease: Targets for Potential Treatments." Translational Neurodegeneration 6(1). doi: 10.1186/s40035-017-0099-z.
McFarthing, Kevin, Gary Rafaloff, Marco Baptista, Leah Mursaleen, Rosie Fuest, Richard K. Wyse, and Simon R. W. Stott. 2022. "Parkinson's Disease Drug Therapies in the Clinical Trial Pipeline: 2022 Update." Journal of Parkinson's Disease 12(4):1073-82. doi: 10.3233/JPD-229002.
Micsonai, András, Frank Wien, Linda Kernya, Young Ho Lee, Yuji Goto, Matthieu Réfrégiers, and József Kardos. 2015. "Accurate Secondary Structure Prediction and Fold Recognition for Circular Dichroism Spectroscopy." Proceedings of the National Academy of Sciences of the United States of America 112(24):E3095-3103. doi: 10.1073/pnas.1500851112.
Mirecka, Ewa A., Hamed Shaykhalishahi, Aziz Gauhar, 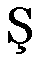 erife Akgül, Justin Lecher, Dieter Willbold, Matthias Stoldt, and Wolfgang Hoyer. 2014. "Sequestration of a β-Hairpin for Control of α-Synuclein Aggregation." *Angewandte Chemie - International Edition* 53(16):4227-30. doi: 10.1002/anie.201309001.
Pagano, Gennaro, Kirsten I. Taylor, Judith Anzures-Cabrera, Maddalena Marchesi, Tanya Simuni, Kenneth Marek, Ronald B. Postuma, Nicola Pavese, Fabrizio Stocchi, Jean-Philippe Azulay, Brit Mollenhauer, Lydia López-Manzanares, David S. Russell, James T. Boyd, Anthony P. Nicholas, Maria R. Luquin, Robert A. Hauser, Thomas Gasser, Werner Poewe, Benedicte Ricci, Anne Boulay, Annamarie Vogt, Frank G. Boess, Juergen Dukart, Giulia D'Urso, Rebecca Finch, Stefano Zanigni, Annabelle Monnet, Nathalie Pross, Andrea Hahn, Hanno Svoboda, Markus Britschgi, Florian Lipsmeier, Ekaterina Volkova-Volkmar, Michael Lindemann, Sebastian Dziadek, Stefan Holiga, Daria Rukina, Thomas Kustermann, Geoffrey A. Kerchner, Paulo Fontoura, Daniel Umbricht, Rachelle Doody, Tania Nikolcheva, and Azad Bonni. 2022. "Trial of Prasinezumab in Early-Stage Parkinson's Disease." New England Journal of Medicine 387(5):421-32. doi: 10.1056/nejmoa2202867.
Paslawski, W., N. Lorenzen, and D. E. Otzen. 2016. "Formation and Characterization of α-Synuclein Oligomers." Pp. 133-50 in Protein Amyloid Aggregation. Methods in Molecular Biology . Vol. 1345, edited by D. Eliezer. New York: Humana Press. doi: 10.1007/978-1-4939-2978-8_9
Paslawski, Wojciech, Justyna Zareba-Paslawska, Xiaoqun Zhang, Katharina Hölzl, Henrik Wadensten, Mohammadreza Shariatgorji, Shorena Janelidze, Oskar Hansson, Lars Forsgren, Per E. Andrén, and Per Svenningsson. 2019. "a-Synuclein-lipoprotein Interactions and Elevated ApoE Level in Cerebrospinal Fluid from Parkinson's Disease Patients." Proceedings of the National Academy of Sciences of the United States of America 116(30):15226-35. doi: 10.1073/pnas.1821409116. doi: 10.1073/pnas.1821409116
Schenk, Dale B., Martin Koller, Daniel K. Ness, Sue G. Griffith, Michael Grundman, Wagner Zago, Jay Soto, George Atiee, Susanne Ostrowitzki, and Gene G. Kinney. 2017. "First-in-Human Assessment of PRX002, an Anti-α-Synuclein Monoclonal Antibody, in Healthy Volunteers." Movement Disorders 32(2):211-18. doi: 10.1002/mds.26878.
Stahl, Stefan, Torbjörn Gräslund, Amelie Eriksson Karlström, Fredrik Y. Frejd, Per Åke Nygren, and John Löfblom. 2017. "Affibody Molecules in Biotechnological and Medical Applications." Trends in Biotechnology 35(8):691-712. doi: 10.1016/j.tibtech.2017.04.007
Zhong, Xiaotian, and Aaron M. D'Antona. 2021. "Recent Advances in the Molecular Design and Applications of Multispecific Biotherapeutics." Antibodies 10(13). doi: 10.3390/antib10020013

## Claims

1. An alpha synuclein-binding polypeptide comprising a first and a second subunit and a linker linking the C-terminus of the first subunit to the N-terminus of the second subunit, wherein:
the first subunit comprises the amino acid sequence 
GX3X4X5X6X7X8X9X10X11X12X13X14X15X16CX18FIX21X22X23X24X25X26X27X28X29X30AX32
X33X34X35X36X37X38X39X40X41X42X43X44;
 and
the second subunit comprises the amino acid sequence 
X62GX64X65VX67X68X69X70X71X72X73X74X75X76CX78FX80X81X82X83X84X85X86X87X88X89
X90AX92X93LX95X96X97X98X99X100X101X102X103X104,
and wherein
X₃ is G, R, P or K,
X₄ is E, D, N or Q,
X₅ is any amino acid except C,
X₆ is any amino acid except C,
X₇ is any amino acid except C,
X₈ is L or M,
X₉ is P, T, R, L, Y, Gor H,
X₁₀ is N, E, D or Q,
X₁₁ is L, A, I, V or G,
X₁₂ is N or K,
X₁₃ is A or P,
X₁₄ is D, H, S, A or E,
X₁₅ is Q, D, N or E,
X₁₆ is L or I,
X₁₈ is A, I, V, G or L,
X₂₁ is R, Q, N, H or K,
X₂₂ is S, T, I, L or V,
X₂₃ is L, A or V,
X₂₄ is E, Q, D or N,
X₂₅ is D or E,
X₂₆ is D or E,
X₂₇ is P, R, Q, E, S or G,
X₂₈ is S or T,
X₂₉ is Q or N,
X₃₀ is S, Q, E, N or P,
X₃₂ is N or Q,
X₃₃ is L or I,
X₃₄ is L or V,
X₃₅ is A, I or L,
X₃₆ is E or D,
X₃₇ is A, I or L,
X₃₈ is K or R,
X₃₉ is K or R,
X₄₀ is L, A, I or V,
X₄₁ is N or Q,
X₄₂ is D or E,
X₄₃ is A, I or L,
X₄₄ is Q or N,
X₆₂ is G or P,
X₆₄ is E or D,
X₆₅ is any amino acid except C,
X₆₇ is any amino acid except C,
X₆₈ is L or Y,
X₆₉ is P, L, T, R, L, Y or H,
X₇₀ is N, E, D or Q,
X₇₁ is L, A, I, V or G,
X₇₂ is N or K,
X₇₃ is A or P,
X₇₄ is D, H, S or A,
X₇₅ is Q or N,
X₇₆ is W or L,
X₇₈ is A, I, V, G or L,
X₈₀ is L, W, F, I or M,
X₈₁ is R, Q, N, H or K,
X₈₂ is S, T, I, L or V,
X₈₃ is L or A,
X₈₄ is E, Q, D or N,
X₈₅ is D or E,
X₈₆ is D or E,
X₈₇ is P, R, Q, E, S or G,
X₈₈ is S or T,
X₈₉ is Q or N,
X₉₀ is S, Q, E, N or P,
X₉₂ is N or Q,
X₉₃ is L or I,
X₉₅ is A, I or L,
X₉₆ is E or D,
X₉₇ is A, I or L,
X₉₈ is K or R,
X₉₉ is K or R,
X₁₀₀ is L, A, I or V,
X₁₀₁ is N or Q,
X₁₀₂ is D or E,
X₁₀₃ is A, I or L, and
X₁₀₄ is Q or N.

2. The polypeptide of claim 1, wherein
X₃ is G or R, preferably G,
X₄ is E,
X₅ is R, P, L, T or E,
X₆ is I, V or F,
X₇ is Y, V, SorT,
X₈ is L,
X₉ is P,
X₁₀ is N,
X₁₁ is L,
X₁₂ is N,
X₁₃ is A,
X₁₄ is D,
X₁₅ is Q,
X₁₆ is L,
X₁₈ is A or L, preferably A,
X₂₁ is R,
X₂₂ is S,
X₂₃ is A or L, preferably A,
X₂₄ is E or Q, preferably E,
X₂₅ is D,
X₂₆ is D,
X₂₇ is P,
X₂₈ is S,
X₂₉ is Q,
X₃₀ is S,
X₃₂ is N,
X₃₃ is L,
X₃₄ is L,
X₃₅ is A,
X₃₆ is E,
X₃₇ is A,
X₃₈ is K,
X₃₉ is K,
X₄₀ is L,
X₄₁ is N,
X₄₂ is D,
X₄₃ is A,
X₄₄ is Q,
X₆₂ is G,
X₆₄ is E,
X₆₅ is P, R, T, I, V or A, preferably P, R or T,
X₆₇ is Y, L, W or M, preferably Y or L,
X₆₈ is L,
X₆₉ is P,
X₇₀ is N,
X₇₁ is L,
X₇₂ is N,
X₇₃ is P,
X₇₄ is D,
X₇₅ is Q,
X₇₆ is W,
X₇₈ is A,
X₈₀ is L, W, For M, preferably W or F, most preferably W,
X₈₁ is R,
X₈₂ is S,
X₈₃ is L,
X₈₄ is E,
X₈₅ is D,
X₈₆ is D,
X₈₇ is P,
X₈₈ is S,
X₈₉ is Q,
X₉₀ is S,
X₉₂ is N,
X₉₃ is L,
X₉₅ is A,
X₉₆ is E,
X₉₇ is A,
X₉₈ is K,
X₉₉ is K,
X₁₀₀ is L,
X₁₀₁ is N,
X₁₀₂ is D,
X₁₀₃ is A, and/or
X₁₀₄ is Q.

3. The polypeptide of claim 1 or 2, wherein X₅X₆X₇X₈ is RIVL, RIYL, PVYL, LVYL, RVYM, TFSL, RFYL, RFTL or EVYL, preferably RIYL, PVYL, LVYL, TFSL or EVYL, more preferably RIYL, LVYL or TFSL.

4. The polypeptide of any one of the preceding claims, wherein X₆₅VX₆₇X₆₈ is PVYL, TVLL or RVYL, such as PVYL or TVLL.

5. The polypeptide of any one of the preceding claims, wherein X₁₂X₁₃X₁₄X₁₅X₁₆CX₁₈ is NADQLCA.

6. The polypeptide of any one of the preceding claims, wherein X₇₆ and X₈₀ are both W.

7. The polypeptide of any one of the preceding claims, wherein the first subunit comprises or consists of:
(i) a sequence selected from SEQ ID NO:1, SEQ ID NO:2, SEQ ID NO:3, SEQ ID NO:4, SEQ ID NO:5, SEQ ID NO:6, SEQ ID NO:8, SEQ ID NO:9, SEQ ID NO:10 and SEQ ID NO:11; or
(ii) a sequence having at least 90%, such as at least 95%, identity to any one of SEQ ID NO:1, SEQ ID NO:2, SEQ ID NO:3, SEQ ID NO:4, SEQ ID NO:5, SEQ ID NO:6, SEQ ID NO:8, SEQ ID NO:9, SEQ ID NO:10 and SEQ ID NO:11.

8. The polypeptide of any one of the preceding claims, wherein the first subunit comprises or consists of:
(iii) a sequence selected from SEQ ID NO:12, SEQ ID NO:13, SEQ ID NO:14, SEQ ID NO:15, SEQ ID NO:16, SEQ ID NO:17, SEQ ID NO:19, SEQ ID NO:20, SEQ ID NO:21 and SEQ ID NO:22; or
(iv) a sequence having at least 90%, such as at least 95%, identity to any one of SEQ ID NO:12, SEQ ID NO:13, SEQ ID NO:14, SEQ ID NO:15, SEQ ID NO:16, SEQ ID NO:17, SEQ ID NO:19, SEQ ID NO:20, SEQ ID NO:21 and SEQ ID NO:22.

9. The polypeptide of any one of the preceding claims, wherein the first subunit comprises or consists of:
(v) a sequence selected from SEQ ID NO:23, SEQ ID NO:24, SEQ ID NO:25, SEQ ID NO:26, SEQ ID NO:27, SEQ ID NO:28, SEQ ID NO:30, SEQ ID NO:31, SEQ ID NO:32 and SEQ ID NO:33; or
(vi) a sequence having at least 90%, such as at least 95%, identity to any one of SEQ ID NO:23, SEQ ID NO:24, SEQ ID NO:25, SEQ ID NO:26, SEQ ID NO:27, SEQ ID NO:28, SEQ ID NO:30, SEQ ID NO:31, SEQ ID NO:32 and SEQ ID NO:33.

10. The polypeptide of any one of the preceding claims, wherein the first subunit comprises or consists of:
(vii) a sequence selected from SEQ ID NO:34, SEQ ID NO:35, SEQ ID NO:36, SEQ ID NO:37, SEQ ID NO:38, SEQ ID NO:39, SEQ ID NO:41, SEQ ID NO:42, SEQ ID NO:43 and SEQ ID NO:44; or
(viii) a sequence having at least 90%, such as at least 95%, identity to any one of SEQ ID NO:34, SEQ ID NO:35, SEQ ID NO:36, SEQ ID NO:37, SEQ ID NO:38, SEQ ID NO:39, SEQ ID NO:41, SEQ ID NO:42, SEQ ID NO:43 and SEQ ID NO:44.

11. The polypeptide of any one of the preceding claims, wherein the linker comprises at least eight amino acid residues, such as 8-30 amino acid residues, such as 8-25 amino acid residues, such as 10-25 amino acid residues.

12. A fusion protein comprising a polypeptide according to any one of the preceding claims and a half-life-extending region, such as an Fc-binding region or an albuminbinding region (ABR).

13. The polypeptide of fusion protein of any one of the preceding claims for use as a medicament.

14. A pharmaceutical composition comprising the polypeptide of fusion protein of any one of the preceding claims.

15. The polypeptide, fusion protein or pharmaceutical composition of any one of the preceding claims for use in a method of treatment of Parkinson's disease or Lewy body dementia.
